# EUROPEAN PATENT SPECIFICATION

(11) **EP 1 508 575 B1**
(45) Date of publication and mention of the grant of the patent: **13.06.2007**
(21) Application number: 03102619.8
(22) Date of filing: 21.08.2003
(51) Int. Cl.: C07K 14/575, A61K 38/22

(54) **Novel leptin antagonist**
Neuer Leptin-Antagonist
Nouvel antagoniste de leptine

(43) Date of publication of application: 23.02.2005
(73) Proprietor: Vlaams Interuniversitair Instituut voor Biotechnologie vzw., 9052 Zwijnaarde (BE)
(72) Inventor: Tavernier, Jan, 9860 Balegem (BE); Peelman, Frank, 9050 Gentbrugge (BE)

(56) References cited:
- WO-A-97/20933
- US-B1- 6 471 956
- VERPLOEGEN S A ET AL: "A human leptin mutant induces weight gain in normal mice." FEBS LETTERS. NETHERLANDS 24 MAR 1997, vol. 405, no. 2, 24 March 1997 (1997-03-24), pages 237-240, XP002062174 ISSN: 0014-5793
- RAVER NINA ET AL: "Comparison of R128Q mutations in human, ovine, and chicken leptins" GENERAL AND COMPARATIVE ENDOCRINOLOGY, vol. 126, no. 1, March 2002 (2002-03), pages 52-58, XP002267945 ISSN: 0016-6480

## Description

The present invention relates to an antagonist or inhibitor of leptin signalling via the leptin receptor. The leptin antagonist binds to the leptin receptor, but is unable to induce JAK-STAT signal transduction via the leptin receptor. By binding to the leptin receptor, the leptin antagonist impairs binding of leptin to the leptin receptor and blocks leptin signalling.

After cleavage of its 21 amino acids signal peptide (Cohen et al., 1996), mature human leptin is secreted as a 146 amino acid protein, with a typical type II interleukin structure, consisting of a bundle of 4 helices (helix 1-4), with an up-up-down-down topology (Zhang et al., 1997). Leptin is secreted into the bloodstream primarily by adipocytes, and blood concentrations of leptin correlate with white adipose tissue mass. Leptin acts as an energy homeostasis hormone, regulating energy expenditure and food intake.
Leptin does so by binding to the leptin receptor in certain areas in the hypothalamus, which leads to phosphorylation of STAT molecules that subsequently migrate to the cell nucleus and induce transcription of different genes.
In addition to its adipostatic function, leptin has many other functions: it can induce proliferation, differentiation and functional activation of haemopoietic cells (Gainsford et al., 1996), and induces angiogenesis (Sierra-Honigmann et al., 1998).
Leptin also interacts with the immune and inflammatory responses (Loffreda et al., 1998).
Leptin levels are acutely increased by inflammatory stimuli and by pro-inflammatory cytokines TNF-α and IL-1 (Grunfeld et al., 1996). Leptin itself regulates the production of several cytokines in vitro, regulates the T helper (Th1/Th2) balance, and can up-regulate inflammatory responses (Loffreda et al., 1998; Faggioni et al., 1998; Lord et al., 1998).

The human leptin receptor is expressed at the cell surface of many different tissues. At least six different splice variants of the human leptin receptor were found at present. The longest isoform of the human leptin receptor consists of 1162 amino acids, with an extracellular region between residues 1 and 840, a transmembrane region between residues-841 and 863 and an intracellular region between residues 864 and 1162.
The extracellular part of the human leptin receptor contains at least 7 structural domains (Fong et al., 1998).
Domain 1 (residue 62-178) and 2 (residue 235-328) have a fibronectin type III fold and together form a cytokine receptor module (CRM), named CRM1.
Domain 3 (residue 329 - 427) has a Immunoglobulin type fold.
Domain 4 (residue 428 - 535) and 5 (residue 536 - 635) also have a fibronectine type III fold and together form a second cytokine receptor module (CRM), named CRM2.

Domain 6 and 7 have a fibronectin type III domain structure.
Like all members of the class I cytokine receptor family, the leptin receptor has no intrinsic kinase activity, and uses a cytoplasmic associated Janus kinase (JAK2 in case of the leptin receptor) for intracellular signalling (Ghilardi et al., 1997). In a generally accepted model, leptin binding leads to formation of a receptor complex, allowing activation of JAK2 by cross-phosphorylation. Activated JAK2 then rapidly phosphorylates several tyrosine residues in the cytosolic domain of the leptin receptor. These phosphorylated tyrosine residues provide docking sites for SH2 containing signalling molecules. In the mouse leptin receptor, tyrosine 1138 serves as a binding site for signal transducer and activator of transcription 3 (STAT3)(Baumann et al., 1996). STAT3 itself is a substrate for JAK2 and dimerizes upon phosphorylation, translocates to the nucleus and modulates transcription of target genes.

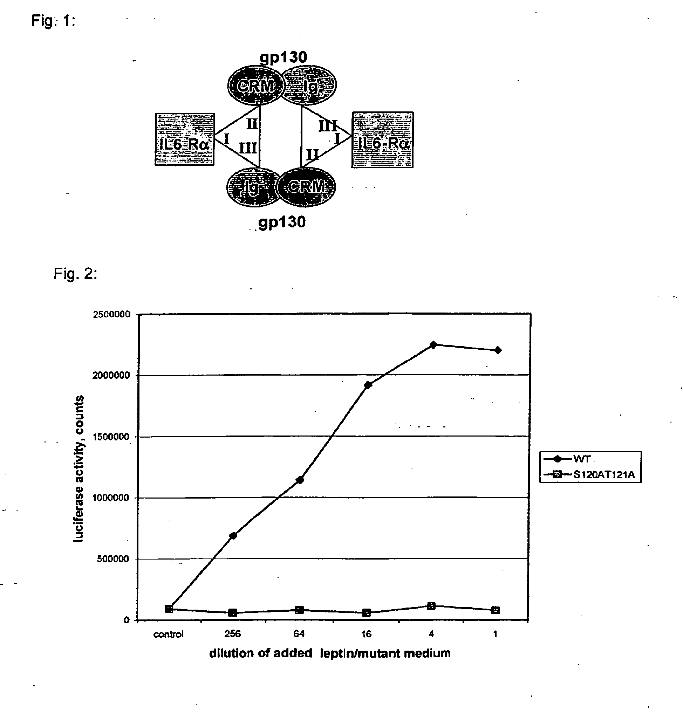

The leptin receptor shows the highest sequence similarity with the cytokine receptors of the IL-6 family and with the Granulocyte Colony-Stimulating Factor (G-CSF) Receptor. FSSP (Holm and Sander, 1997) structural similarity searches reveal that leptin shows the highest structural similarity with the cytokines of the IL-6 family and G-CSF, and to a lesser extent with other long chain cytokines, such as the growth hormone and placental lactogen. The crystal structure of the Kaposi's sarcoma-associated herpesvirus IL-6 (vIL6, viral IL-6) in a 2:2 complex with the three N-terminal extracellular domains of human gp130 reveals two binding sites, binding site II and binding site III, for interaction between vIL6 and gp130 (Chow et al, 2001). Binding site II, consisting of residues in helices 1 and 3 of vIL6, interacts with the cytokine receptor module (CRM) of gp130. Binding site III in vIL6 consists of residues in the N-terminus of helix 4, in the loop connecting helix 3 and 4 and in the loop connecting helix 1 and 2, and interacts with the Immunoglobulin-like domain of gp130. Corresponding site II and III residues were identified in other members of the IL-6 family of cytokines by site directed mutagenesis: human IL-6, human IL-11, Leukemia inhibitory factor (LIF), oncostatin M (OSM) and Ciliary neurotrophic factor (CNTF) (Kalai et al., 1997; Savino et al., 1993; DiMarco et al., 1996; Hudson et al., 1996; Inoue et al., 1995; Barton et al., 1999; Bravo and Heath, 2000).
IL-6 contains a third binding site, binding site I, for interaction with the IL-6 α receptor. Human IL-6 forms a hexameric 2:2:2 complex with its gp130 and IL-6 α receptor chains: each IL-6 molecule binds two gp130 molecules by its site II and III binding sites, and one IL-6 receptor α subunit (IL-6Rα) by its binding site I (figure 1). (Boulanger et al., 2003).

Activation of the leptin receptor by binding of leptin plays a role in several physiological processes. Several variant and mutant forms of leptin have been described, that can be used in different applications. WO02062833 describes modified leptin polypeptides that are substantially non-immunogenic or less immunogenic than any non-modified counterpart when used in vivo. These polypeptides can be administered to humans of therapeutic use. WO9700319 discloses chimeric leptin polypeptides comprising leptin or a mutant or variant thereof fused to a human immunoglobulin domain. These chimeric derivatives have prolonged clearing rates and may be useful in the treatment or prophylaxis of obesity, or diseases and conditions associated with obesity such as atherosclerosis, hypertension and type II diabetes. WO9720933 discloses mutational variants of the mammalian leptin. These molecules can serve as agonist or antagonist of the wild type leptin; their capacity to induce the signaling pathway upon binding of the receptor varies for the different muteins. WO9812224 describe the use of fragments, derived from leptin, as leptin antagonist, especially for treating type II diabetes.

Verploegen et al (Febs Letters 405 (1997): 237) disclose a leptin antagonist with the amino acid substitution R128Q which is capable of binding the leptin receptor with the same affinity as wild-type leptin without inducing intracellular signalling.

There is, however, still need for a leptin mutant that is able to bind to the receptor, with a similar or higher affinity as the wild type leptin, but without remaining signaling activity. Such leptin mutant would be a powerful antagonist and can be used to treat leptin-mediated diseases.

Surprisingly, we found that leptin, similar to IL-6 and G-CSF, has a binding site II and III, which are both involved in binding to the leptin receptor. As is the case for G-CSF and for members of the IL-6 family, binding site II consists of residues in helix 1 (residues 4 - 26) and 3 (residues 71 - 93). These residues are involved in high affinity binding to the CRM2 module of the leptin receptor, and mutations in binding site II of mouse leptin decrease or even abolish the affinity of the mutant leptin to the mouse leptin receptor. Due to the decreased binding, these mutants show a decreased induction of JAK-STAT signalling via the leptin receptor.
Binding site III consists of residues in the loop connecting helix 1 and 2 (residues 27 - 50) and of residues at the N-terminus of helix 4 (residues 105 - 122). Mutations in this binding site do not affect the high affinity binding to the leptin receptor or to the CRM2 module, but still show a decreased induction of JAK-STAT signalling via the leptin receptor. The S120AT121A mutation in mouse leptin binding site III is unable to induce any JAK-STAT reporter activity via the mouse leptin receptor, although the binding affinity of this mutant for the leptin receptor or for CRM2 of the mouse leptin receptor is similar to that of wild type leptin.
Even more surprisingly, we found that the S120AT121A mouse leptin mutant can inhibit the binding of wild type leptin to its receptor, and can inhibit the JAK-STAT signal induced by wild-type leptin. This is because the S120AT121A leptin mutant shows an intact binding site II, and avidly binds to CRM2 of the leptin receptor, thus competing for binding of wild type leptin. Unlike wild type leptin, however, binding of the S120AT121A mutant does not induce JAK-STAT signalling.

A first aspect of the invention is a leptin antagonist of polypeptidic nature, comprising SEQ ID N°1. Preferably, said leptin antagonist is comprising SEQ ID N°2 or 3, even more preferably said leptin antagonist is comprising SEQ ID N° 4. Preferably, said leptin antagonist is binding to the leptin receptor, without inducing the signaling pathway. A preferred embodiment is a leptin antagonist according to the invention, capable of binding the leptin receptor without inducing the signaling pathway, whereby said antagonist is mutated in amino acid 120 and/or 121 of SEQ ID N°5. Preferably, amino acid 120 or amino acid 121 are mutated into an alanine. Even more preferably, both amino acids are changed into alanine.
Another preferred embodiment is a leptin antagonist of polypeptidic nature, capable of binding the leptin receptor without inducing the signaling pathway, comprising SEQ ID N°5 or a functional fragment. A functional fragment as used here is a fragment that still can bind to the leptin receptor. Chemical modifications of the antagonist can also be considered. Chemical modifications of polypeptides are known to the person skilled in the art, and include but are not limited to natural occurring modifications such as glycosylation, phosphorylation, ubiquitinilation and artificial modifications such as PEGylation. Preferably, said chemical modification is increasing the half-life time of the polypeptide. Even more preferably, said chemical modification is PEGylation. Said functional fragment can be used as such, or it may be fused to another polypeptide. In the latter case, the fusion polypeptide has preferably an increased antagonistic capacity. Possible fusion partners are, as a non- limiting example, polypeptides that increase the half-life time of the polypeptide in vivo. Such polypeptides are disclosed, amongst others, in WO9700319. Alternatively, the fusion partner may capture leptin itself, thereby increasing the antagonistic activity. Polypeptides that capture leptin are, as a non-limiting example, leptin antibodies preferably single chain antibodies, or a domain of the leptin receptor that is binding leptin.
A preferred fusion partner for the antagonist it the immunoglobulin type fold domain of the leptin receptor. This domain is involved in leptin dependent oligomerisation of the leptin receptor, and subsequent signaling. Fusion of this domain to the antagonist will therefore give an increased antagonistic affect, by disturbing the leptin induced oligomerization of the receptor.
Another aspect of the invention is the use of a leptin antagonist according to the invention for the preparation of a medicament to treat T-cell mediated immune and/or autoimmune diseases. Indeed, leptin enhances T cell mediated immune responses, by signalling through the long form of the leptin receptor on CD4+ T lymphocytes (Lord et al., 1998). Leptin shifts the T-cell responses towards a Th1 type, with increased secretion of pro-inflammatory cytokines IL-2 and interferon-γ, and decreased IL-4 production. A leptin antagonist can therefore be used for modifying/attenuating the T-cell immune responses, with use as a drug for the treatment of T-cell mediated (auto-) immune diseases.
Still another aspect of the invention is the use of an antagonist according to the invention for the preparation of a medicament to treat intestinal inflammation diseases. Preferably, said intestinal inflammation diseases are selected from the group consisting of Crohn's disease, ulcerative colitis and intestinal infectious diseases. In experimental mouse model systems, where chronic and acute colitis is induced by dextran sulfate sodium or trinitrobenzene sulfonic acid, leptin deficient mice (ob/ob mice), show a 72% reduction of colitis severity and a similar decrease of pro-inflammatory cytokines in the intestine, compared to wild type mice (Siegmund et al., 2002). Administration of leptin in the leptin deficient mice abolishes the resistance against experimentally induced colitis (Siegmund et al., 2002). Administration of *Clostridium difficile* toxin A induces severe colitis in mice. Leptin deficient (ob/ob) mice, as well as leptin receptor deficient (db/db) mice are partially protected against the toxin A- induced intestinal secretion and inflammation (Mykoniatis et al., 2003). In ob/ob, but not in db/db mice, leptin administration reverses the protection against toxin A-induced intestinal secretion and inflammation (Mykoniatis et al., 2003). A leptin antagonist can therefore be used as a drug for treatment of intestinal inflammation diseases, such as Crohn's disease, ulcerative colitis and intestinal infectious diseases.
In case of intestinal inflammation diseases, a preferred delivery method for the leptin antagonist according to the invention is an in vivo delivery system, as described in WO9714806. Therefore, another aspect of the invention is a lactic acid bacterium, producing a leptin antagonist according to the invention. Preferably, said lactic acid bacterium is a Lactobacillus, even more preferably said lactic acid bacterium is a Lactococcus.
A further aspect of the invention is the use of a leptin antagonist according to the invention for the preparation of a medicament to treat rheumathoid arthritis. Administration of methylated BSA in the knees of mice leads to the development of Antigen-induced arthritis. As compared to wild type mice, leptin deficient (ob/ob) mice and leptin receptor deficient (db/db) mice develop less severe arthritis, with decreased IL-1β and TNF-α in the knee synovial fluid, decreased serum levels of anti-methylated BSA antibodies and a decreased antigen-specific T cell proliferative response (Busso et al., 2002). A leptin antagonist can therefore be used as a drug for treatment of rheumatoid arthritis.
Still another aspect of the invention is the use of a leptin antagonist according to the invention for the preparation of a medicament to treat multiple sclerosis. The clinical onset of experimental autoimmune encephalomyelitis (EAE), a mouse model for multiple sclerosis, in disease-susceptible C57BL/6J(H-2b) and SJL/J(H-2s) mice is preceded by an increase in serum leptin concentrations (Sanna et al., 2003). This increase is correlated with disease susceptibility. Acute starvation, which reduces serum leptin levels, delays disease onset and attenuates the EAE symptoms. Leptin-deficient C57BL/6J-ob/ob mice are resistant against EAE, while this resistance is abolished by administration of leptin (Matarese et al., 2001). These data strongly indicate that leptin is a required factor for development of EAE, and thus, probably for multiple sclerosis. A leptin antagonist can therefore be used as a drug for treatment of multiple sclerosis.
A further aspect of the invention is the use of a leptin antagonist according to the invention for the preparation of a medicament to treat Type 1 diabetes. Type 1 diabetes is an autoimmune disease, in which the pancreatic β-cells are destroyed by inflammatory processes. In the non-obese diabetic (NOD) mouse, an animal model for type 1 diabetes, an increased serum level of leptin precedes the diabetes in susceptible females, while injection of leptin accelerates the autoimmune destruction of the pancreatic β-cells (Matarese et al., 2002). A leptin antagonist can be used as a drug for preventing/treating type 1 diabetes.
A further aspect of the invention is the use of a leptin antagonist according to the invention for the preparation of a medicament to prevent and/or treat diseases characterized by T-cell mediated hepatotoxicity. Leptin deficient ob/ob mice are protected from T cell-mediated hepatitis, experimentally induced with ConA or Pseudomonas aeruginosa exotoxin A (Faggioni et al., 2000). Injection of leptin in the leptin deficient mice restores the hepatotoxicity of these compounds (Faggioni et al., 2000). A leptin antagonist can therefore be used for treatment and/ or prevention of diseases characterized by T-cell mediated hepatotoxicity. Still another aspect of the invention is a pharmaceutical composition, comprising a leptin antagonist according to the invention, optionally with a pharmaceutical acceptable excipient. Suitable excipients are known to the person skilled in the art, and are inherently non-toxic and nontherapeutic. Excipients may be, as a non limiting example, Ringer's solution, dextrose solution or Hank's solution. Non aqueous solutions such as fixed oils and ethyl oleate may also
be used. A preferred excipient is 5% dextrose in saline. The excipient may contain minor amounts of additives such as substances that enhance isotonicity and chemical stability, including buffers and preservatives.

### BRIEF DESCRIPTION OF THE FIGURES

**Figure 1:** composition of the 2 :2 :2 IL-6: gp130 : IL-6Rα complex.
**Figure 2:** Activation of luciferase as reporter by WT leptin and the S120AT121A mutant.
**Figure 3:** Competition of Leptin mutants for binding of SEAP-Leptin to LepR CRM2. Inset: binding of SEAP-Leptin to LepR CRM2 in the presence of undiluted medium containing WT leptin or the R20N or S120AT121A mutant leptin.

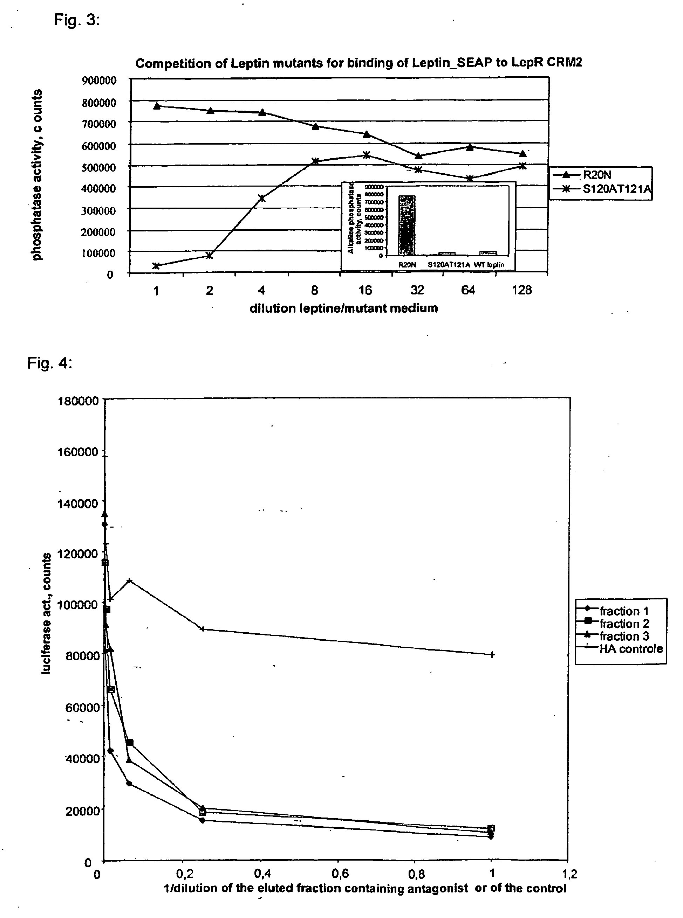

**Figure 4:** Incubation of the transfected HEK293T cells with the wt HA-mouse leptin induces luciferase activity. Co-incubation with the HA-tagged S120AT121A mouse leptin, purified on the anti-HA affinity column, leads to strong inhibition of the leptin-induced luciferase activity, and the luciferase activity drops to the level of non-stimulated cells. 50 µl of the HEK293T cells were incubated with WT leptin and 50 µl of different dilutions of fractions 1 (◇), 2 (□), and 3(Δ) eluted from the anti HA column with 1mg/ml HA peptide in equilibration buffer. As a control (+), 50 µl of the HEK293T cells were incubated with WT leptin and 50 µl of different dilutions of the elution buffer (1mg/ml HA peptide in equilibration buffer).

### EXAMPLES

### Example 1: Detection of binding site II and III in human and mouse leptin by structural superposition.

FSSP structural similarity searches reveal that leptin shows the highest structural similarity with the cytokines of the IL-6 family and G-CSF, and to a minor extent with other long chain cytokines, such as the growth hormone and placental lactogen.
The crystal structures of human leptin (1ax8), human CNTF (1cnt), human IL-6 (1alu), bovine G-CSF(1bgc), vIL6 (1i1r), ovine placental lactogen (1f6f), murine LIF (11ki) and human OSM (1evs) were superposed, using the FSSP and Prosup algorithms. Human leptin residues overlapping with binding site II or III residues in the other cytokines were considered as possible binding site II or III residues in human leptin.
A homology model was built for murine leptin, by replacing non-identical residues in human leptin structure by the optimal rotamer of the corresponding residue in mouse leptin, followed by energy minimization, using moe and the charmm22 forcefield. Residues aligning with the possible binding site II or III residues in human leptin were considered as possible binding site II or III residues in human leptin. Solvent-exposed residues in the predicted binding site II and III were mutated in the pMET7-SlgK-HA-mLep expression vector, and the mutant leptin was expressed in COS-1 cells.

### Example 2: Generation of the mouse leptin expression vector pMet7-SlgK-HA-mouse leptin

The pCDM8 mleptin-AP vector is containing the mouse leptin sequence, followed by the human alkaline phosphatase sequence. The wild type (wt) mouse leptin sequence was isolated from this vector by PCR, using the 5' forward oligomeric primer 5'-GCGTCCGGAATCCAGAAAGTCCAGGATG-3', containing a BspEl restriction site, and the 3' reverse primer 5'-CGCTCTAGATTAGCATTCAGGGCTAACATCC-3' , containing an Xbal restriction site. The pMET7-SlgK-HA-LRlo plasmid contains the SlgK signal peptide, followed by the HA tag sequence and the mouse leptin receptor sequence. The leptin receptor sequence was excised from this vector, using the BspEl and Xbal restriction enzymes, and the mouse leptin sequence was ligated into this opened vector. The resulting vector, pMET7-SlgK-HA-mLep, allows the expression of a fusion protein, consisting of the SlgK signal peptide, followed by the HA-tag sequence, followed by a 4 amino acid GGSG linker, followed by amino acids 3 to 146 of mouse leptin. Upon expression in eukaryotic cells, the SlgK signal peptide is cleaved off and the HA-tagged protein is secreted in the medium.

Amino acid sequence of SlgK-HA-mouse leptin: the arrow indicates the predicted cleavage site of the SlgK signal peptide, the numbers above the sequence indicate the residue numbers in mouse leptin:

### Example 3: Generation of mouse leptin mutants

The mouse leptin S120AT121A mutation was introduced in the pMET7-SlgK-HA-mLep by PCR using primers O-1821 and O-1822.
The mouse leptin R20N mutation was introduced in the pMET7-SlgK-HA-mLep by PCR using primers O-1701 and O-1702
The mouse leptin L13N mutation was introduced in the pMET7-SlgK-HA-mLep by PCR using primers O-1769 and O-1770.

| **Oligo** | **Specification** | **Sequence** |
|---|---|---|
| O-1701 | Mutation R20N in mouse leptin | CAAGACCATTGTCACCAACATTAATGACATTTCACACACG |
| O-1702 | Mutation R20N in mouse leptin | CGTGTGTGAAATGTCATTAATGTTGGTGACAATGGTCTTG |
| O-1821 | Mutation S120AT121A in mouse leptin | GGAAGCCTCACTCTACGCCGCGGAGGTGGTGGCTTTG |
| O-1822 | Mutation S120AT121A in mouse leptin | CAAAGCCACCACCTCCGCGGCGTAGAGTGAGGCTTCC |
| O-1769 | Mutation L13N in mouse leptin | GGATGACACCAAAACCAACATCAAGACAATTGTCACCAGGATC |
| O-1770 | Mutation L13N in mouse leptin | GATCCTGGTGACAATTGTCTTGATGTTGGTTTTGGTGTCATCC |

All mutations were introduced using the QuickChange method (Stratagene) according to the manufacturer's specifications.

Amino acid sequence of the mouse leptin mutants: vertical arrows indicate the position of the mutations, the numbers above the sequence indicate the residue numbers in mouse leptin:

### Example 4: Expression of the HA-tagged mouse leptin and HA-tagged mouse leptin mutants in COS-1 cells

COS-1 cells were seeded at 4 x 10⁵ cells/well in 6-well plates, and grown overnight.
The cells were then transfected using the polyethyleneimine (PEI) transfection method with the pMet7-SlgK-HA-mLep or the R20N, L13N or S120AT121A mutants in this vector.
The medium was replaced after 4 hours transfection, and cells were incubated overnight, after which the medium was replaced by 2 ml OPTI-MEM medium (Gibco-BRL). After another 72 hours, the OPTI-MEM medium containing the secreted HA-tagged leptin or HA-tagged leptin mutant was collected, and cells were removed by centrifugation.

### Example 5: Purification of HA-tagged S120AT121A mouse leptin

COS-1 cells were seeded at 8 x 10⁶ cells per 175 cm² flask in DMEM medium. Ten 175 cm² flasks were transfected with the S120AT121A pMET7-SlgK-HA-mLep mutant by transfection using polyethyleneimine. After 4 hours, the medium was replaced by fresh DMEM medium, and cells were grown overnight. The medium was then replaced by 50 ml OPTI-MEM, and cells were incubated in this medium for another 72 hours. The medium with the secreted S120AT121A HA-tagged mouse leptin was collected and filtered through a 0.22 µm filter, and complete (Roche Diagnostics) protease inhibitor was added.
The S120AT121A HA-tagged mouse leptin was purified on a 1ml anti-HA affinity column (Roche diagnostics). The medium was loaded at a flow rate of 0.3 ml/min. The column was washed with 25 ml equilibration buffer (20 mM Tris/HCl, pH 7.5, 100 mM NaCl, 0.1 mM EDTA) + 0.05% Tween-20, followed by 10 ml equilibration buffer without Tween-20. The S120AT121A HA-mouse leptin was eluted with HA peptide (1 mg/ml) in equilibration buffer.

### Example 6: The S120AT121A mouse leptin mutant does not induce JAK-STAT signalling of the mouse leptin receptor

HEK293T cells were seeded in 6 well plates at 4 x 10⁵ cells/well and grown for 20 hours. The cells were then transfected with the pMET7-mLRlo and pXP2d2-rPAP1 plasmids, using the standard calcium phosphate precipitation technique. One day after transfection, the cells were washed with phosphate buffered saline, and cultured in DMEM medium supplemented with 10% foetal calf serum and 50 µg/ml gentamycin. Two days after transfection, the cells were dissociated with cell dissociation buffer (Invitrogen) and resuspended in 2ml of DMEM medium supplemented with 10% foetal calf serum and 50 µg/ml gentamycin. 50 µl of the cell suspension was seeded in each well of a black 96-well plate (Costar). In each well, 50 µl of the appropriate dilution of medium containing either HA-tagged mouse leptin, R20N mouse leptin, L13N mouse leptin or S120AT121A mouse leptin was added.

The pMET7-mLRlo plasmid encodes the long form of the mouse leptin receptor, with a C-terminal myc tag. The pXP2d2-rPAP1 plasmid encodes the luciferase gene, under control of the STAT3-dependent rat pancreatitis-associated protein-1 promotor.
Incubation of the transfected HEK293T cells with wt HA-tagged mouse leptin leads to concentration-dependent luciferase activity. Incubation of the transfected HEK293T cells with the R20N mutant HA-tagged mouse leptin, the L13N mutant HA-tagged mouse leptin or the S120AT121A mouse leptin does not lead to an appreciable increase of luciferase activity in the transfected cells.

### Example 7: The S120AT121A mouse leptin inhibits binding of wt leptin to the leptin receptor

8 x 10⁶ COS-1 cells were seeded in a 175 cm2 flask, and grown overnight in DMEM medium supplemented with 10% foetal calf serum and 50 µg/ml gentamycin.

The cells were transfected with the pMET7-mLRCRM2-his6 using the PEI transfection method. pMET7-mLRCRM2-his6 encodes amino acids 407- 604, corresponding to the CRM2 module of the murine leptin receptor, followed by a C-terminal his6 tag. After 4 hours, the medium was replaced by fresh DMEM medium supplemented with 10% foetal calf serum and 50 µg/ml gentamycin, and cells were grown overnight. The medium was then replaced by 50 ml OPTI-MEM, and cells were incubated in this medium for another 72 hours. The medium with the secreted His-tagged CRM2 leptin was collected and cells were removed by centrifugation. This medium is referred to as CRM2-his medium.
8 x 10⁶ COS -1 cells were seeded in a 175 cm² flask, and grown overnight in DMEM supplemented with 10% foetal calf serum and 50 µg/ml gentamycin.
The cells were transfected with the pCDM8 AP-mLep plasmid using the PEI transfection method. The pCDM8 AP-mLep plasmid allows expression of a fusion protein, consisting of murine leptin linked to the C-terminus of human secreted alkaline phosphatase (SEAP). After 4 hours, the medium was replaced by fresh DMEM medium, and cells were grown overnight. The medium was then replaced by 50 ml OPTI-MEM, and cells were incubated in this medium for another 72 hours. The medium with the secreted SEAP-leptin was collected and cells were removed by centrifugation. This medium is referred to as SEAP-leptin medium.
Maxisorp plates (Nunc) were coated overnight at 6 °C with 0.25 µg/ml anti-His5 antibody (Qiagen). Plates were washed four times with phosphate buffered saline pH 7.5 containing 0.1 % Tween 20. The free protein binding sites on the plates were blocked by incubation with 1% casein in phosphate buffered saline pH 7.5 at 37 °C for two hours. Plates were washed four times with phosphate buffered saline pH 7.5 containing 0.1 % Tween 20. The plates were incubated for 2 hours at 37 °C with the undiluted medium containing the his-tagged CRM2 of the murine leptin receptor. Plates were washed four times with phosphate buffered saline pH 7.5 containing 0.1 % Tween 20. The plates were then incubated with SEAP-leptin medium and different concentrations of either HA-tagged mouse leptin or S120AT121A leptin antagonist. Addition of S120AT121A mouse leptin or WT leptin, but not of mutant R20N inhibits the binding of the SEAP-leptin to the his-tagged CRM2 of the murine leptin receptor. The results are shown in Figure 3.

### Example 8: The S120AT121A mouse leptin mutant inhibits the JAK-STAT signalling of the mouse leptin receptor

HEK 293T cells were seeded in 6 well plates at 4 x 10⁵ cells/well and grown for 20 hours. The cells were then transfected with the pMET7-mLRlo and pXP2d2-rPAP1 plasmids, using the standard calcium phosphate precipitation technique. One day after transfection, the cells were washed with phosphate buffered saline, and cultured in DMEM medium supplemented with 10% foetal calf serum and 50 µg/ml gentamycin. Two days after transfection, the cells were dissociated with cell dissociation buffer (Invitrogen) and resuspended in 2ml of DMEM medium, supplemented with 10% foetal calf serum and 50 µg/ml gentamycin. 50 µl of the cell suspension was seeded in each well of a black 96-well plate (Costar). OPTI-MEM medium, containing wt HA-tagged leptin (see example 4) was diluted 16 fold in OPT-MEM. 50 µl of this diluted medium was added to the cells, together with 50 µl of dilution of anti-HA purified (see example 5) HA tagged S120AT121A leptin. The cells were then incubated overnight. The medium was removed and the cells were incubated with 50 µl of lysis buffer (25 mM Tris, pH 7.8; 2 mM EDTA; 2 mM DTT; 10% glycerol; 1% Triton X-100) for 10 minutes. 35 µl of luciferase substrate buffer (20 mM Tricine; 1.07 mM (MgCO₃)₄Mg(OH)₂.5H₂O; 2.67 mM MgSO₄.7H₂O; 0.1 mM EDTA; 33.3 mM DTT; 270 mM Coenzyme A; 470 mM Luciferin; 530 mM ATP; final pH 7.8) was then added to the lysate, and the light emission was measured in a TopCount Chemiluminescence Counter (Packard).

The results are shown in Figure 4. Incubation of the transfected HEK293T cells with the wt HA-tagged mouse leptin induces luciferase activity. Co-incubation with the S120AT121A mutant inhibits the leptin-induced luciferase activity. This inhibitory effect is not seen with the L13N nor the R20N mutants.

### REFERENCES

Barton, V.A., Hudson, K.R. and Heath, J.K. (1999) Identification of three distinct receptor binding sites of murine interleukin-11. J. Biol. Chem., 274, 5755-5761.
Baumann, H., Morella, K.K., White, D.W., Dembski, M., Bailon, P.S., Kim, H., Lai, C.-F. and Tartaglia, L.A. (1996) The full-length leptin receptor has signalling capabilities of interleukin 6-type cytokine receptors. Proc. Natl. Acad. Sci., 93, 8374-8378.
Boulanger, M.J., Chow, D.-C., Brevnova, E.E. and Garcia, K.C. (2003) Hexameric structure and assembly of the interleukin-6/ IL-6 α-receptor / gp130 complex. Science, 300, 2101-2104.
Bravo, J., and Heath, J.K. (2000) Receptor recognition by gp130 cytokines. EMBO J., 19, 2399-2411.
Busso, N., So, A., Chobaz-Péclat V., Morard, C., Martinez-Soria E., Talabot-Ayer D. and Gabay C. (2002) Leptin signalling deficiency impairs humoral and cellular immune responses and attenuates experimental arthritis. J. Immunol., 168, 875-882.
Chow, D.-C., He, X.-L., Snow, A.L., Rose-John, S. and Garcia, K.C. (2001) Structure of an extracellular gp130 cytokine receptor signalling complex. Science, 291, 2150-2155.
Cohen, S.L., Halaas, J.L., Friedman, J.M., Chait, B.T., Bennett, L., Chang, D., Hecht, R. and Collins, F. (1996) Human leptin characterization. Nature. 382, 589.
Di Marco, A.; Gloaguen, I., Graziani, R., Paonessa, G., Saggio, I., Hudson, K.R., and Laufer, R. (1996) Identification of Ciliary neurotrophic factor (CNTF) residues essential for leukemia inhibitory factor receptor binding and generation of CNTF receptor antagonists. Proc. Natl. Acad. Sci. USA, 93, 9247-9252.
Faggioni, R., Fantuzzi, G., Fuller, J., Dinarello, C.A., Feingold, K.R., Grunfeld, C. (1998) IL-1 beta mediates leptin induction during inflammation. Am J Physiol., 274, R204-208
Fong, T.M., Huang,R.-R.C., Tota, M.R., Mao, C., Smith, T., Varnerin,J., Karpitskiy, V.V., Krause, J.E., Van Der Ploeg, L.H.T. (1998) Localization of Leptin Binding Domain in the leptin receptor. Molecular Pharmacology, 53, 234-240.
Gainsford, T., Willson, T.A., Metcalf, D., Handman, E., McFarlane, C., Ng, A., Nicola, N.A., Alexander, W.S., Hilton, D.J. (1996) Leptin can induce proliferation, differentiation, and functional activation of hemopoietic cells. Proc. Natl. Acad. Sci. USA, 93, 14564-14568.
Ghilardi, N., Skoda, R.C. 1997 The leptin receptor activates janus kinase 2 and signals for proliferation in a factor-dependent cell line. Mol Endocrinol, 11, 393-9.
Grunfeld, C., Zhao, C., Fuller, J., Pollack, A., Moser, A., Friedman, J., Feingold, K.R. (1996) Endotoxin and cytokines induce expression of leptin, the ob gene product, in hamsters. J Clin Invest., 97, 2152-2157.
Holm, L. and Sander, C. (1997). Dali/FSSP classification of three-dimensional protein folds. Nucleic Acids Res. , 25, 231-234.
Hudson, K.R., Vernallis, A.B., and Heath J.K. (1996) Characterization of the receptor binding sites of human leukemia inhibitory factor and creation of antagonists. J. Biol. Chem. 271, 11971-11978.
Inoue M., Nakayam, C., Kikuchi, K., Kimura, T., Ishige, Y., Ito, A., Kanaoka, M. and Noguchi, H. (1995) D1 cap region involved in the receptor recognition and neuronal cell survival activity of human Ciliary neurotrophic factor. Proc. Natl. Acad. Sci. USA, 92, 8579-8583.
Kalai, M.; Montero-Julian., F., Grötzinger, J. ; Fontaine, V., Vandenbussche, P., Deschuyteneer, R., Wollmer, A., Brailly, F., and Content, J. (1997) Analysis of the human Interleukin-6/ Human Interleukin-6 Receptor binding interface at the amino acid level: proposed mechanism of interaction. Blood, 89, 1319-1333.
Loffreda, S., Yang, S.Q., Lin, H.Z., Karp, C.L., Brengman, M.L., Wang, D.J., Klein, AS., Bulkley, G.B., Bao, C., Noble, P.W., Lane, M.D., Diehl, A.M. (1998) Leptin regulates proinflammatory immune responses. Faseb J., 12, 57-65.
Lord, G.M., Matarese, G., Howard, J.K., Baker, R.J., Bloom, S.R., Lechler, R.I. (1998) Leptin modulates the T-cell immune response and reverses starvation-induced immunosuppression. Nature, 394, 897-901.
Matarese, G., Di Giacomo, A., Sanna, V., Lord, G.M., Howard, J.K., DiTuoro, A., Bloom, S.R., Lechler, R.I., Zappacosta, S. and Fontana, S. (2001) requirement for leptin in the induction and progression of autoimmune encephalomyelitis. J. Immunology, 166, 5909-5916.
Matarese, G., Sanna, V., Lechler, R.I., Sarvetnick, N., Fontana, S., Zappacosta, S., and La Cava, A. (2002) Leptin accelerates autoimmune diabetes in female NOD mice. Diabetes, 51, 1356-1361.
Mykoniatis A., Anton, P.M., WLK, M., Wang, C.C., Ungsunan, L., Blüher, S., Venihaki, M., simeonidis, S., Zacks, J., Zhao, D., Sougioultzis, S., Karalis, K., Mantzoros, C., and Pothoulakis, C. (2003) Leptin mediates Clostridium Difficile Toxin A- induced enteritis in mice. Gastroenterology, 124, 683-691.
Sanna, V., Di Giacomo, A., La Cava, A., Lechler, R.I., Fontana, S., Zappacosta, S., and Matarese, G. (2003) Leptin surge precedes onset of autoimmune encephalomyelitis and correlates with development of pathogenic T cell responses. J. Clin. Invest., 111, 241-250.
Savino, R., Lahm, A., Giogio, M., Cabibbo, A., Tramontano, A. and Ciliberto, G. (1993) Saturation mutagenesis of the human interleukin 6 receptor-binding site: Implications for its three-dimensional structure. Proc. Natl. Acad. Sci. USA, 90, 4067-4071.
Siegmund, B., Lehr, H.A., and Fantuzzi, G. (2002) Leptin: A pivotal mediator of intestinal inflammation in mice. Gastroenterology, 122, 2011-2025.
Sierra-Honigmann, M.R., Nath, A.K., Murakami, C., García-Cardeña, G., Papapetropoulos, A., Sessa, W.C., Madge, L.A., Schechner, J.S., Scwabb, M.B., Polverini, P.J., Flores-Riveros, J.R. (1998) Biological action of leptin as an angiogenic factor. Science, 281, 1683-1686.
Zhang, F., Basinski, M.B., Beals, J.M., Briggs, S.L., Churgay, L.M., Clawson, D.K., DiMarchi, R.D., Furman, T.C., Hale, J.E., Hsiung, H.M., Schoner, B.E., Smith, D.P., Zhang, X.Y., Wery, J.P., Schevitz, R.W. (1997) Crystal structure of the obese protein leptin-E100. Nature, 387, 206-209.

## Claims

1. A leptin antagonist, comprising SEQ ID N°1.

2. A leptin antagonist according to claim 1, comprising a sequence selected from the group of SEQ ID N°2, SEQ ID N°3 and SEQ ID N°4.

3. A leptin antagonist, capable of binding the leptin receptor without inducing the signaling pathway, comprising SEQ ID N°5 or a functional fragment thereof.

4. A leptin antagonist according to claim 3, whereby said antagonist comprises an A residue at position 120 and/or 121 of SEQ ID N° 5.

5. The use of a leptin antagonist according to any of the preceding claims for the preparation of a medicament to treat T-cell mediated immune and/or autoimmune diseases.

6. The use of a leptin antagonist according to any of the claims 1-4 for the preparation of a medicament to treat intestinal inflammation diseases.

7. The use of a leptin antagonist according to claim 6, whereby said intestinal inflammation disease is selected from the group Crohn's disease, ulcerative colitis and intestinal infectious disease.

8. The use of a leptin antagonist according to any of the claims 1-4 for the preparation of a medicament to treat rheumatoid arthritis.

9. The use of a leptin antagonist according to any of the claims 1-4 for the preparation of a medicament to treat multiple sclerosis.

10. The use of a leptin antagonist according to any of the claims 1-4 for the preparation of a medicament to treat Type 1 diabetes.

11. The use of a leptin antagonist according to any of the claims 1-4for the preparation of a medicament to treat T-cell mediated hepatoxicity.

12. A pharmaceutical composition, comprising a leptin antagonist according to any of the claims 1-4.

## Patentansprüche

1. Leptinantagonist, der SEQ ID Nr. 1 umfasst.

2. Leptinantagonist nach Anspruch 1, der eine Sequenz umfasst, die aus der Gruppe von SEQ ID Nr. 2, SEQ ID Nr. 3 und SEQ ID Nr. 4 ausgewählt ist.

3. Leptinantagonist, der den Leptinrezeptor binden kann, ohne den Signalstoff-Stoffwechselweg zu induzieren, und der SEQ ID Nr. 5 oder ein funktionelles Fragment davon umfasst.

4. Leptinantagonist nach Anspruch 3, wobei der Antagonist einen A-Rest an Position 120 und/oder 121 von SEQ ID Nr. 5 umfasst.

5. Verwendung eines Leptinantagonisten nach einem der vorhergehenden Ansprüche zur Herstellung eines Arzneimittels, um T-zellvermittelte Immun- oder Autoimmunerkrankungen zu behandeln.

6. Verwendung eines Leptinantagonisten nach einem der Ansprüche 1 - 4 zur Herstellung eines Arzneimittels, um Darmentzündungserkrankungen zu behandeln.

7. Verwendung eines Leptinantagonisten nach Anspruch 6, wobei die Darmentzündungserkrankung aus der Gruppe Morbus Crohn, Colitis ulcerosa und Darminfektionserkrankung ausgewählt ist.

8. Verwendung eines Leptinantagonisten nach einem der Ansprüche 1 - 4 zur Herstellung eines Arzneimittels, um rheumatoide Arthritis zu behandeln.

9. Verwendung eines Leptinantagonisten nach einem der Ansprüche 1 - 4 zur Herstellung eines Arzneimittels, um multiple Sklerose zu behandeln.

10. Verwendung eines Leptinantagonisten nach einem der Ansprüche 1-4 zur Herstellung eines Arzneimittels, um Typ-1-Diabetes zu behandeln.

11. Verwendung eines Leptinantagonisten nach einem der Ansprüche 1-4 zur Herstellung eines Arzneimittels, um T-zellvermittelte Hepatotoxizität zu behandeln.

12. Pharmazeutische Zusammensetzung, die einen Leptinantagonisten nach einem der Ansprüche 1 - 4 umfasst.

## Revendications

1. Antagoniste de leptine, comprenant SEQ ID N° 1.

2. Antagoniste de leptine selon la revendication 1, comprenant une séquence sélectionnée parmi le groupe de SEQ ID N° 2, SEQ ID N° 3 et SEQ ID N° 4.

3. Antagoniste de leptine, capable de se lier au récepteur de leptine sans induire la voie de signalisation, comprenant SEQ ID N° 5 ou un fragment fonctionnel de celle-ci.

4. Antagoniste de leptine selon la revendication 3, moyennant lequel ledit antagoniste comprend un résidu A en position 120 et/ou 121 de SEQ ID N° 5.

5. Utilisation d'un antagoniste de leptine selon l'une quelconque des revendications précédentes pour la préparation d'un médicament pour traiter des maladies immunes et/ou autoimmunes induites par les lymphocytes T.

6. Utilisation d'un antagoniste de leptine selon l'une quelconque des revendications 1 à 4 pour la préparation d'un médicament pour traiter des maladies inflammatoires intestinales.

7. Utilisation d'un antagoniste de leptine selon la revendication 6, moyennant lequel ladite maladie inflammatoire intestinale est sélectionnée parmi le groupe de la maladie de Crohn, la recto-colite hémorragique et la maladie infectieuse intestinale.

8. Utilisation d'un antagoniste de leptine selon l'une quelconque des revendications 1 à 4 pour la préparation d'un médicament pour traiter la polyarthrite rhumatoïde.

9. Utilisation d'un antagoniste de leptine selon l'une quelconque des revendications 1 à 4 pour la préparation d'un médicament pour traiter la sclérose en plaques.

10. Utilisation d'un antagoniste de leptine selon l'une quelconque des revendications 1 à 4 pour la préparation d'un médicament pour traiter le diabète de type 1.

11. Utilisation d'un antagoniste de leptine selon l'une quelconque des revendications 1 à 4 pour la préparation d'un médicament pour traiter l'hépatoxicité induite par les lymphocytes T.

12. Composition pharmaceutique, comprenant un antagoniste de leptine selon l'une quelconque des revendications 1 à 4.


### SEQUENCE LISTING

<110> VIB vzw
<120> NOVEL LEPTIN ANTAGONIST
<130> JTA -leptant -163
<160> 17
<170> PatentIn version 3.1
<210> 1
   <211> 16
   <212> PRT
   <2.13> Artificial Sequence
<220>
   <223> a leptin antagonist
<220>
   <221> MISC_FEATURE
   <222> (1)..(1)
   <223> X can be any amino acid, but not S
<220>
   <221> MISC_FEATURE
   <222> (2)..(2)
   <223> X can be any amino acid, but not T
<220>
   <221> MISC_FEATURE
   <222> (9)..(9)
   <223> X can be an R or W
<220>
   <221> MISC_FEATURE
   <222> (12)..(12)
   <223> X can be an G, A or R
<220>
   <221> MISC_FEATURE
   <222> (13)..(13)
   <223> X can be an S or A
<400> 1
   Xaa Xaa Glu Val Val Ala Leu Ser Xaa Leu Gln Xaa Xaa Leu Gln Asp
   1               5                   10                  15
<210> 2
   <211> 16
   <212> PRT
   <213> Artificial Sequence
<220>
   <223> a leptin antagonist
<220>
   <221> MISC_FEATURE
   <222> (2).. (2)
   <223> X can be any amino acid, but not T
<220>
   <221> MISC_FEATURE
   <222> (9)..(9)
   <223> X can be an R or W
<220>
   <221> MISC_FEATURE
   <222> (12) . . (12)
   <223> X can be an G, A or R
<220>
   <221> MISC_FEATURE
   <222> (13)..(13)
   <223> X can be an S or A
<400> 2
   Ala Xaa Glu Val Val Ala Leu Ser Xaa Leu Gln Xaa Xaa Leu Gln Asp
   1               5                   10                  15
<210> 3
   <211> 16
   <212> PRT
   <213> Artificial Sequence
<220>
   <223> a leptin antagonist
<220>
   <221> MISC_FEATURE
   <222> (1). . (1)
   <223> X can be any amino acid, but not S
<220>
   <221> MISC_FEATURE
   <222> (9).. (9)
   <223> X can be an R or W
<220>
   <221> MISC_FEATURE
   <222> (12)..(12)
   <223> X can be an G, A or R
<220>
   <221> MISC_FEATURE
   <222> (13)..(13)
   <223> X can be an S or A
<400> 3
   Xaa Ala Glu Val Val Ala Leu Ser Xaa Leu Gln Xaa Xaa Leu Gln Asp
   1               5                   10                  15
<210> 4
   <211> 16
   <212> PRT
   <213> Artificial Sequence
<220>
   <223> a leptin antagonist
<220>
   <221> MISC_FEATURE
   <222> (9)..(9)
   <223> X can be an R or W
<220>
   <221> MISC_FEATURE
   <222> (12). .(12)
   <223> X can be an G, A or R
<220>
   <221> MISC_FEATURE
   <222> (13) .(13)
   <223> X can be an S or A
<400> 4
   Ala Ala Glu Val Val Ala Leu Ser Xaa Leu Gln Xaa Xaa Leu Gln Asp
   1               5                   10                  15
<210> 5
   <211> 146
   <212> PRT
   <213> Artificial Sequence
<220>
   <223> a leptin antagonist
<220>
   <221> MISC_FEATURE
   <222> (120)..(120)
   <223> X can be any amino acid, but not S
<220>
   <221> MISC_FEATURE
   <222> (121)..(121)
   <223> X can be any amino acid, but not T
<400> 5
   Val Pro Ile Gln Lys Val Gln Asp Asp Thr Lys Thr Leu Ile Lys Thr
   1               5                   10                  15
   Ile Val Thr Arg Ile Asn Asp Ile Ser His Thr Gln Ser Val Ser Ser
               20                  25                  30
   Lys Gln Lys Val Thr Gly Leu Asp Phe Ile Pro Gly Leu His Pro Ile
           35                  40                  45
   Leu Thr Leu Ser Lys Met Asp Gln Thr Leu Ala Val Tyr Gln Gln Ile
       50                  55                  60
   Leu Thr Ser Met Pro Ser Arg Asn Val Ile Gln Ile Ser Asn Asp Leu
   65                  70                  75                  80
   Glu Asn Leu Arg Asp Leu Leu His Val Leu Ala Phe Ser Lys Ser Cys
                   85                  90                  95
   His Leu Pro Trp Ala Ser Gly Leu Glu Thr Leu Asp Ser Leu Gly Gly
               100                 105                 110
   Val Leu Glu Ala Ser Gly Tyr Xaa Xaa Glu Val Val Ala Leu Ser Arg
           115                 120                 125
   Leu Gln Gly Ser Leu Gln Asp Met Leu Trp Gln Leu Asp Leu Ser Pro
       130                 135                 140
   Gly Cys
   145
<210> 6
   <211> 28
   <212> DNA
   <213> Artificial Sequence
<220>
   <223> forward oligomeric primer used in example 2
<400> 6
   gcgtccggaa tccagaaagt ccaggatg 28
<210> 7
   <211> 31
   <212> DNA
   <213> Artificial Sequence
<220>
   <223> reverse primer used in example 2
<400> 7
   cgctctagat tagcattcag ggctaacatc c 31
<210> 8
   <211> 178
   <212> PRT
   <213> Mus musculus
<220>
   <221> MISC_FEATURE
   <223> SlgK-HA-mouse leptin
<400> 8
   Met Glu Thr Asp Thr Leu Leu Leu Trp Val Leu Leu Leu Trp Val Pro
   1               5                   10                  15
   Gly Ser Thr Gly Asp Tyr Pro Tyr Asp Val Pro Asp Tyr Ala Gly Gly
               20                  25                  30
   Ser Gly Ile Gln Lys Val Gln Asp Asp Thr Lys Thr Leu Ile Lys Thr
           35                  40                  45
   Ile Val Thr Arg Ile Asn Asp Ile Ser His Thr Gln Ser Val Ser Ala
       50                  55                  60
   Lys Gln Arg Val Thr Gly Leu Asp Phe Ile Pro Gly Leu His Pro Ile
   65                  70                  75                  80
   Leu Ser Leu Ser Lys Met Asp Gln Thr Leu Ala Val Tyr Gln Gln Val
                   85                  90                  95
   Leu Thr Ser Leu Pro Ser Gln Asn Val Leu Gln Ile Ala Asn Asp Leu
   100 105 110
   Glu Asn Leu Arg Asp Leu Leu His Leu Leu Ala Phe Ser Lys Ser Cys
   115 120 125
   Ser Leu Pro Gln Thr Ser Gly Leu Gln Lys Pro Glu Ser Leu Asp Gly
   130 135 140
   Val Leu Glu Ala Ser Leu Tyr Ser Thr Glu Val Val Ala Leu Ser Arg
   145 150 155 160
   Leu Gln Gly Ser Leu Gln Asp Ile Leu Gln Gln Leu Asp Val Ser Pro
   165 170 175
   Glu Cys
<210> 9
   <211> 40
   <212> DNA
   <213> Artificial Sequence
<220>
   <223> primer O-1701
<400> 9
   caagaccatt gtcaccaaca ttaatgacat ttcacacacg 40
<210> 10
   <211> 40
   <212> DNA
   <213> Artificial Sequence
<220>
   <223> primer O -1702
<400> 10
   cgtgtgtgaa atgtcattaa tgttggtgac aatggtcttg 40
<210> 11
   <211> 37
   <212> DNA
   <213> Artificial Se quence
<220>
   <223> primer O -1821
<400> 11
   ggaagcctca ctctacgccg cggaggtggt ggctttg **3**7
<210> 12
   <211> 37
   <212> DNA
   <213> Artificial Sequence
<220>
   <223> primer O-1822
<400> 12
   caaagccacc acctccgcgg cgtagagtga ggcttcc 37
<210> 13
   <211> 43
   <212> DNA
   <213> Artificial Sequence
<220>
   <223> primer O -1769
<400> 13
   ggatgacacc aaaaccaaca tcaagacaat tgtcaccagg atc 43
<210> 14
   <211> 43
   <212> DNA
   <213> Artificial Sequence
<220>
   <223> primer O-1770
<400> 14
   gatcctggtg acaattgtct tgatgttggt tttggtgtca tcc 43
<210> 15
   <211> 178
   <212> PRT
   <213> Artificial Sequence
<220>
   <223> mouse leptin mutant R20N
<400> 15
   Met Glu Thr Asp Thr Leu Leu Leu Trp Val Leu Leu Leu Trp Val Pro
   1               5                   10                  15
   Gly Ser Thr Gly Asp Tyr Pro Tyr Asp Val Pro Asp Tyr Ala Gly Gly
               20                  25                  30
   Ser Gly Ile Gln Lys Val Gln Asp Asp Thr Lys Thr Leu Ile Lys Thr
   35 40 45
   Ile Val Thr Asn Ile Asn Asp Ile Ser His Thr Gln Ser Val Ser Ala
   50 55 60
   Lys Gln Arg Val Thr Gly Leu Asp Phe Ile Pro Gly Leu His Pro Ile
   65 70 75 80
   Leu Ser Leu Ser Lys Met Asp Gln Thr Leu Ala Val Tyr Gln Gln Val
   85 90 95
   Leu Thr Ser Leu Pro Ser Gln Asn Val Leu Gln Ile Ala Asn Asp Leu
   100 105 110
   Glu Asn Leu Arg Asp Leu Leu His Leu Leu Ala Phe Ser Lys Ser Cys
   115 120 125
   Ser Leu Pro Gln Thr Ser Gly Leu Gln Lys Pro Glu Ser Leu Asp Gly
   130 135 140
   Val Leu Glu Ala Ser Leu Tyr Ser Thr Glu Val Val Ala Leu Ser Arg
   145 150 155 160
   Leu Gln Gly Ser Leu Gln Asp Ile Leu Gln Gln Leu Asp Val Ser Pro
   165 170 175
   Glu Cys
<210> 16
   <211> 178
   <212> PRT
   <213> Artificial Sequence
<220>
   <223> mouse leptin mutant L13N
<400> 16
   Met Glu Thr Asp Thr Leu Leu Leu Trp Val Leu Leu Leu Trp Val Pro
   1 5 10 15
   Gly Ser Thr Gly Asp Tyr Pro Tyr Asp Val Pro Asp Tyr Ala Gly Gly
   20 25 30
   Ser Gly Ile Gln Lys Val Gln Asp Asp Thr Lys Thr Asn Ile Lys Thr
           35                  40                  45
   Ile Val Thr Arg Ile Asn Asp Ile Ser His Thr Gln Ser Val Ser Ala
       50                  55                  60
   Lys Gln Arg Val Thr Gly Leu Asp Phe Ile Pro Gly Leu His Pro Ile
   65                  70                  75                  80
   Leu Ser Leu Ser Lys Met Asp Gln Thr Leu Ala Val Tyr Gln Gln Val
                   85                  90                  95
   Leu Thr Ser Leu Pro Ser Gln Asn Val Leu Gln Ile Ala Asn Asp Leu
               100                 105                 110
   Glu Asn Leu Arg Asp Leu Leu His Leu Leu Ala Phe Ser Lys Ser Cys
           115                 120                 125
   Ser Leu Pro Gln Thr Ser Gly Leu Gln Lys Pro Glu Ser Leu Asp Gly
       130                 135                 140
   Val Leu Glu Ala Ser Leu Tyr Ser Thr Glu Val Val Ala Leu Ser Arg
   145                 150                 155                 160
   Leu Gln Gly Ser Leu Gln Asp Ile Leu Gln Gln Leu Asp Val Ser Pro
                   165                 170                 175
   Glu Cys
<210> 17
   <211> 178
   <212> PRT
   <213> Artificial Sequence
<220>
   <223> mouse leptin mut ant S120AT121A
<400> 17